# EUROPÄISCHE PATENTSCHRIFT

(11) **EP 1 028 722 B1**
(45) Veröffentlichungstag und Bekanntmachung des Hinweises auf die Patenterteilung: **24.01.2007**
(21) Anmeldenummer: 98939461.4
(22) Anmeldetag: 28.08.1998
(51) Int. Cl.: A61K 31/35, A61K 9/02, A61K 9/48, A61P 33/06

(54) **PHARMAZEUTISCH WIRKSAME ZUSAMMENSETZUNG ENTHALTEND ARTEMISININ UND/ODER EIN DERIVAT VON ARTEMISININ**
PHARMACEUTICALLY ACTIVE COMPOSITION CONTAINING ARTEMISININE AND/OR A DERIVATIVE OF ARTEMISININE
COMPOSITION PHARMACEUTIQUEMENT ACTIVE CONTENANT DE L'ARTEMISINE ET/OU UN DERIVE D'ARTEMISINE

(30) Priorität: 03.11.1997 CH 253597
(43) Veröffentlichungstag der Anmeldung: 23.08.2000
(73) Patentinhaber: Mepha AG, CH-4147 Aesch (CH)
(72) Erfinder: SCHEIWE, Max, Werner, D-79689 Maulburg (DE)
(74) Vertreter: Braun, André jr.
(86) Internationale Anmeldenummer: PCT/CH1998/000372
(87) Internationale Veröffentlichungsnummer: WO 1999/022727

(56) Entgegenhaltungen:
- US-A- 4 920 147

## Beschreibung

Die vorliegende Erfindung betrifft eine pharmazeutisch wirksame Zusammensetzung, welche eine gegen Malariaparasiten wirksame Substanz enthält und eine hohe Aktivität gegen multiresistente Linien von plasmodium falciparum aufweist. Insbesondere betrifft die vorliegende Erfindung eine akzeptable Form von Artemisinin und/oder einem Derivat von Artemisinin, wie beispielsweise Arteäther (engl. arteether), Artemether, Artemisin oder Artesunat, insbesondere Artesunat, für die rektale Verwendung. Artesunat ist ein wasserlösliches Derivat von Artemisinin. Artemisinin (Quinghaosu) wird aus den Blättern des Strauches artemisia annua gewonnen und ist ein natürlich vorkommendes Sesquiterpenlacton mit einer Endoperoxidgruppe. Aufgrund der geringen Wasserlöslichkeit des Naturstoffes Artemisinin, wurde versucht, diesen in verschiedene synthetische Derivate umzuwandeln, um eine verbesserte pharmazeutische Verfügbarkeit zu erreichen. So beschreibt US Patent 4,920,147 Deoxoartemisinin zur Behandlung von Malaria, das sich durch gute Lagerstabilität und Bioverfügbarkeit auszeichnet und hinsichtlich Lagerstabilität und Verweildauer im Blut Artemisinin deutlich überlegen ist. Ein solches Derivat ist auch Artesunat. Artemisinin und Artesunat sind effiziente Wirkstoffe in der Behandlung der Malaria. Artemisinin-Präparate sind derzeit die am schnellsten gegen Malariaparasiten wirkenden Substanzen. Sie zeigen insbesondere eine hohe Aktivität gegen multiresistente Linien von plasmodium falciparum. Auch sind bei der Anwendung beim Menschen nur wenige Nebeneffekte und keine signifikante Toxizität beobachtet worden, obwohl Neurotoxizität im Tier aufgetreten ist. Allerdings ist bisher relativ wenig über die Pharmakogenetik von Artemisinin und Artesunat bekannt geworden, was mit den komplizierten analytischen Verfahren, welche für eine Quantifizierung dieser Arzneimittel im Blut benötigt werden, zusammenhängen mag.

Im Körper werden Artemisinin wie auch Artesunat in Dihydroartemisinin (DHA, Artesol) umgewandelt, welches die eigentliche schizontozide Wirksubstanz darstellt. Damit kann man Artemisinin wie auch Artesunat bezüglich Dihydroartemisinin als Prodrugs bezeichnen.

Artesunat entspricht der Verbindung Dihydroartemisinin-hemisuccinat sowie deren Salzen, insbesondere deren Natriumsalz. Dihydroartemisinin wird chemisch als 3α,12α-Epoxy-3,4,5,5aα, 6,7,8aα,9,10,12β,12a-dodecahydro-10-hydroxy-3β,6α,9β-trimethylpyrano[4,3-j]-1,2-benzodioxepin bezeichnet. Dihydroartemisinin ist auch unter dem Namen Dihydroquinghaosu bekannt. Artesunat bzw. Dihydroartemisinin-hemisuccinat kann man beispielsweise herstellen, indem man Dihydroartemisinin mittels Acylierung in Dihydroartemisinin-hemisuccinat umwandelt. Arteether, Artemether und Artemisin sind an sich bekannt.

Artemisinin, Artesunat, Dihydroartemisinin und deren Derivate werden insbesondere in China und in Vietnam hergestellt und beispielsweise in Tablettenform verkauft. Bei der schweren eventuell cerebralen Malana, die durch Plasmodium falciparum verursacht wird, sind die Patienten häufig bewusstlos und eine perorale Behandlung ist nicht möglich. Die parenterale Verabreichung, beispielsweise von Chinin, kommt nur im Spital in Frage. In solchen Fällen kann Artesunat in rektal applizierbarer Form lebensrettend eingesetzt werden, und zwar auf der ganzen Welt und praktisch in jeder Situation. Ein weiterer Vorteil der rektalen Form ist die Anwendung bei Kindern, wo die perorale Anwendung oft mit Schluckproblemen verbunden ist. Rektale Formen von Artesunat können auch bei andern Malariaarten, z.B. VIVAX, erfolgreich verabreicht werden.

In Afrika sterben jährlich etwa eine Million Kinder an Malaria. Zwar sind bereits Tabletten und Injektionslösungen, die als Pulverzubereitung vorliegen, im Handel erhältlich, nicht aber Suppositorien. Dies hängt vor allem damit zusammen, dass Artesunat in üblichen Suppositorienrezepturen instabil ist, insbesondere bei erhöhter Temperatur. Zahlreiche Gebiete, beispielsweise subtropische und tropische Regionen, haben oft ein überdurchschnittlich warmes Klima und somit auch überdurchschnittlich hohe Temperaturen. Im weiteren sind Suppositorien an sich in der Regel bei erhöhter Aussentemperatur instabil, da sie bei ca. 37°C schmelzen sollen.

Aufgabe der vorliegenden Erfindung ist es, eine rektale Arzneimittelform zu finden, welche Artemisinin und/oder ein Derivat von Artemisinin, wie beispielsweise Arteether, Artemether, Artemisin und/oder Artesunat, insbesondere Artesunat als Wirkstoff enthält, wobei diese Form eine ausreichende Stabilität des Wirkstoffes bei angemessener Lagerdauer aufweist und eine genügende Menge an Wirkstoff enthält, so dass eine sichere Bekämpfung der Plasmodium-Erreger im Blut ermöglicht wird. Rektale Formen sind beispielsweise Suppositorien, Rektalschäume, Klistiere oder Rektalkapseln. Im folgenden wird vorwiegend der Ausdruck Artesunat stellvertretend für Artemisinin und dessen Derivate verwendet.

Die übliche Vorgabe für Herstellung von Rektalkapseln besteht darin, dass in jedem Falle oberflächenaktive Stoffe, insbesondere Netzmittel (Tenside), aber auch Emulgatoren und gegebenenfalls waschaktive Stoffe anwesend sein müssen, um eine ausreichende Bioverfügbarkeit des Wirkstoffes durch Verbesserung der Spreitung im Rektum zu gewährleisten. Die Untersuchungen zeigten jedoch, dass bereits nach kurzer Lagerdauer eine hochgradige Instabilität solcher Artesunat Formulierungen vorliegt.

Oberflächenaktive Stoffe werden entsprechend deren "Hydrophilic-Lipophilic-Balance" mittels deren HLB-Werte in Antischaummittel (HLB-Werte von 1-3), Emulgatoren (HLB-Werte von 4-6 und 8-18), Netzmittel (HLB-Werte von 7-9), waschaktive Stoffe (HLB-Werte von 13-15) und Solubilisatoren (HLB-Werte von 10-18) unterteilt. Dabei überschneiden sich die Grenzen bezüglich der Wirkung teilweise und sind fliessend. Eine Verbindung kann, je nach der Zusammensetzung in welcher sie sich befindet, trotz ihres HLB-Werts eine ungenügende oder allenfalls auch keine der zugeordneten Wirkung zeigen oder beispielsweise sowohl als Emulgator als auch als Netzmittel wirken. Verbindungen, welche als Antischaummittel, Emulgatoren, Netzmittel, waschaktive Stoffe und Solubilisatoren bezeichnet werden, sind an sich bekannt.

Es wurde nun überraschenderweise gefunden, dass eine Zusammensetzung, welche im wesentlichen frei ist von Verbindungen mit einem HLB-Wert im Bereich von 7-9.9, und den Wirkstoff Artemisinin und/oder ein Derivat von Artemisinin, insbesondere Artesunat, in einem gegenüber dem Wirkstoff inerten Trägermaterial enthält, nicht nur eine galenisch stabile Formulierung ergibt, welche die geforderten Stabilitätskriterien bezüglich der chemischen Beständigkeit des Wirkstoffes erfüllt, sondern auch eine Bioverfügbarkeit des Wirkstoffes aufweist, die innerhalb der international anerkannten Grenzen liegt, welche für die vergleichende Bioverfügbarkeit angewendet werden.

Im Sinne der vorliegenden Erfindung bedeutet die Formulierung "im wesentlichen frei" von Verbindungen mit einem HLB-Wert im Bereich von 7-9.9, dass die gegebenenfalls vorhandene Verbindung mit einem HLB-Wert im Bereich von 7-9.9, vorzugsweise eine Verbindung mit einem HLB-Wert im Bereich von 7-9, in der Zusammensetzung eine Konzentration aufweist, welche höchstens 2 Gewichtsprozent, bezogen auf das Gewicht des pharmazeutisch aktiven Wirkstoffes, beträgt. Meist bevorzugt ist, dass keine Verbindung mit einem HLB-Wert im Bereich von 7-9.9, vorzugsweise keine Verbindung mit einem HLB-Wert im Bereich von 7-9, in der Zusammensetzung anwesend ist.

Die vorliegende Erfindung ist in den Patentansprüchen definiert. Insbesondere betrifft die Erfindung eine pharmazeutisch wirksame Zusammensetzung in Form von rektal anwendbaren Suppositorien, Rektalschäumen, Klistiere oder Rektalkapseln mit deutlich verbesserter Lagerstabilität, welche dadurch gekennzeichnet ist, dass:
(i) diese Zusammensetzung den Wirkstoff Artemisinin, Arteether, Artemether, Artemisin und/oder Artesunat, in einem gegenüber dem Wirkstoff inerten Trägermaterial enthält, wobei
(ii) dieses Trägermaterial eine Verbindung oder ein Gemisch von Verbindungen darstellt, ausgewählt aus der Gruppe umfassend: pflanzliche Wachse und Wachse tierischen Ursprungs mit Schmelzpunkten im Bereich von 47°C bis 88°C; Paraffinwachse mit Schmelzpunkten im Bereich von 47°C bis 65°C; mikrokristalline Wachse mit Schmelzpunkten im Bereich von 54°C und 105°C; Fette mit Schmelzpunkten im Bereich von 28°C bis 45°C und Öle mit Schmelzpunkten im Bereich von 0°C bis 20°C;
(iii) das Gewichtsverhältnis des Wirkstoffs zum Trägermaterial 5% bis 15%, bezogen auf das Gesamtgewicht des Trägermaterials, beträgt, und
(iv) das Trägermaterial Verbindungen mit einem HLB-Wert von 7-9.9 in einer Konzentration von höchstens 2 Gew.-%, bezogen auf das Gewicht des Wirkstoffes, enthält.

Unter dem Begriff Artemisinin und Derivate von Artemisinin sind insbesondere Arteether, Artemether, Artemisin und/oder Artesunat, vorzugsweise Artesunat zu verstehen.

Die vorliegende Erfindung betrifft im weiteren die Verwendung der erfindungsgemässen Zusammensetzung für die Herstellung von pharmazeutischen rektal anwendbaren Formen. Solche rektal anwendbare Formen sind vorzugsweise Suppositorien, Rektalschäume, Klistiere und Rektalkapseln.

Die vorliegende Erfindung betrifft im weiteren rektale Formen, vorzugsweise Suppositorien, Rektalschäume, Klistiere oder Rektalkapseln, vorzugsweise Suppositorien und Rektalkapseln, welche eine erfindungsgemässe Zusammensetzung enthalten. Bevorzugt sind Rektalkapseln.

Die vorliegende Erfindung betrifft im weiteren Suppositorien, welche dadurch gekennzeichnet sind, dass diese aus einer Hartgelatinekapsel oder einer Weichgelatinekapsel bestehen, welche die erfindungsgemässe Zusammensetzung in einer pharmazeutisch wirksamen Menge enthalten, wobei die Hartgelatinekapsel oder die Weichgelatinekapsel mit einem gleitfähigen Überzug versehen ist, welcher aus an sich bekannten Überzugsmaterialien für Rektalkapseln besteht. Bevorzugt sind Suppositorien, welche aus einer Weichgelatinekapsel bestehen. Die vorliegende Erfindung betrifft auch die Verwendung dieser Suppositorien für die Bekämpfung von Malariaparasiten und multiresistenten Linien von plasmodium falciparum.

Vorzugsweise ist die erfindungsgemässe Zusammensetzung im wesentlichen auch frei von Verbindungen, welche einen HLB-Wert im Bereich von 4-6.9 und 10-13 aufweisen. Das heisst, dass eine solche gegebenenfalls in der Zusammensetzung anwesende Verbindung eine Konzentration von höchstens 2 Gewichtsprozent, bezogen auf das Gewicht des pharmazeutisch aktiven Wirkstoffes, aufweist. Meist bevorzugt ist in der Zusammensetzung keine Verbindung anwesend, welche einen HLB-Wert.im Bereich von 4-6 und 10-13, und vorzugsweise im Bereich von 4-6.9 und 10-13, aufweist.

Vorzugsweise ist die erfindungsgemässe Zusammensetzung im wesentlichen auch frei von Verbindungen, welche einen HLB-Wert im Bereich von 13.1-15 aufweisen. Das heisst, dass eine solche gegebenenfalls in der Zusammensetzung anwesende Verbindung eine Konzentration von höchstens 2 Gewichtsprozent, bezogen auf das Gewicht des pharmazeutisch aktiven Wirkstoffes, aufweist. Meist bevorzugt ist in der Zusammensetzung keine Verbindung anwesend, welche einen HLB-Wert im Bereich von 13-15, und vorzugsweise im Bereich von 13.1-15, aufweist.

Bevorzugt sind Suppositorien und Rektalkapseln. Suppositorien sollen bei etwa 37°C schmelzen. In heissen Ländern, wo die durchschnittliche Temperatur im Mittel bereits bei 37°C oder oberhalb dieser Temperatur liegt, kommen deshalb Rektalkapseln zur Anwendung, während in Breitengraden, wo die Temperaturen im Mittel unterhalb 37°C liegen, eher Suppositorien verwendet werden.

Artesunat ist Dihydroartemisinin-hemisuccinat (C₁₉H₂₈O₈) und entspricht der Formel

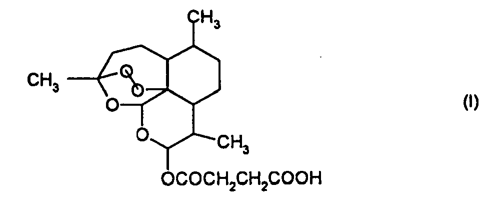

Netzmittel können anionisch, kationisch, amphoter oder nichtionogen sein. Übliche Netzmittel haben einen HLB-Wert von etwa 7-9 und sind beispielsweise anionaktive Netzmittel wie Alkali- oder Ammoniumsalze von ungesättigten Fettsäuren, insbesondere Alkalialkylsulfate, z.B. Natriumdodecylsulfat, Natriumlaurylsulfat, Natriumcetylstearylsulfat, Natriumdocusat; Alkali- und Erdalkalisalze von Alkyl- oder Arylalkylsulfonaten und Salze der Gallensäure wie Natriumcholat sowie saure Saponine. Kationische Netzmittel sind beispielsweise quaternäre Ammoniumverbindungen. Amphotere Netzmittel sind beispielsweise Lecithine und Betainderivate. Nichtionogene Netzmittel sind beispielsweise Fettalkohole, Cholesterine, gegebenenfalls in Kombination mit primären Emulgatoren, wie z.B. emulgierender Cetylstearylalkohol (Kombination Natriumcetylstearylsulfat und Cetylstearylalkohol) oder Cetomacrogol emulsifying wachs, Cholesterin, partielle Fettsäureester des Glycerins, wie Glycerinmonofettsäureester, z.B. Glycerinmonostearat, gegebenenfalls in Kombination mit hydrophilen Emulgatoren. Partielle Fettsäureester von Sorbitan, oxäthylierte partielle Fettsäureester von Sorbitan, andere partielle Fettsäureester, Fettsäureester von Polyoxyäthylen, Fettalkoholäther des Polyoxyäthylens, Fettsäureester der Saccharose, Fettsäureester des Polyglycerins, Blockcopolymere des Polyoxyäthylens und des Polyoxypropylens. Solche Tenside werden in der Regel in einer Menge von 1 bis 10 Prozent bezogen auf die Füllmasse verwendet. Gemäss der vorliegenden Erfindung werden vorzugsweise keine Netzmittel verwendet.

Gegenüber dem Wirkstoff inerte Trägermaterialien sind insbesondere Wachse, Fette und Öle, sowie Mischungen derselben. Diese können pflanzlichen oder tierischen Ursprungs und auch hydriert sein. Bevorzugt sind Wachse, Fette und Öle pflanzlichen Ursprungs. Ebenso können Paraffinwachse und Paraffinöle verwendet werden.

Wachse sind beispielsweise natürliche Pflanzenwachse, wie Carnaubawachs, Wachse tierischen Ursprungs, wie gelbes oder weisses Bienenwachs, Stearinwachse, mit Schmelzpunkten (bzw. Schmelzpunktintervallen), welche zwischen etwa 47°C und etwa 88°C liegen. Es können aber auch Paraffinwachse verwendet werden, wie beispielsweise Hartparaffine mit Schmelzpunkten zwischen etwa 47°C und etwa 65°C, mikrokristalline Wachse mit Schmelzpunkten zwischen etwa 54°C und etwa 105°C. Solche Wachse verwendet man vorallem als Konsistenzgeber.

Fette sind im allgemeinen Triglyceride mit (C₁₈ - C₂₄)-Fettsäuren und vorwiegend (C₁₈ - C₁₉)-Fettsäuren, welche Schmelzpunkte bzw. Schmelzpunktintervalle aufweisen, welche zwischen etwa 28°C und etwa 45°C liegen. Hartfette in Form von halbsynthetischen Fetten für die Suppositorienherstellung bestehen aus einem Gemisch aus Mono-, Di- und Triglyceriden von gesättigten (C₁₀ - C₁₈-)Fettsäuren. Auch Paraffinfette, das heisst Paraffine, deren Schmelzpunkte bzw. Schmelpunktintervalle in den genannten Bereichen liegen, können verwendet werden. Fette sind das eigentliche Trägermaterial.

Öle sind im allgemeinen mittelkettige Triglyceride mit (C₈ - C₁₆)-Fettsäuren und vorwiegend (C₈ - C₁₂)-Fettsäuren, welche bei Raumtemperatur flüssig sind und Schmelzpunkte bzw. Schmelzpunktintervalle aufweisen, welche zwischen 0°C und 20°C und vorzugsweise bei etwa 0°C bis 10°C liegen. Auch Paraffinöle, das heisst Paraffine, deren Schmelzpunkte bzw. Schmelzpunktintervalle in den genannten Bereichen liegen, können verwendet werden. Öle werden vorallem als Lösungsmittel oder Lösungsvermittler, als Suspendierhilfsstoff oder als Emulgator verwendet.

Solche Fette und/oder Öle können beispielsweise aus pflanzlichen Rohstoffen wie Kokosnüssen, Palmkernen, Oliven, Raps, oder aus tierischen Rohstoffen wie Rindertalg, Schweineschmalz oder Walfischspeck gewonnen und gegebenenfalls hydriert werden

Das Gewichtsverhältnis des pharmazeutischen Wirkstoffs zum Trägermaterial ist vorzugsweise 5% bis 15%, vorzugsweise 7% bis 12% und vorzugsweise etwa 8.5% bis 9.5% bezogen auf das Gesamtgewicht des Trägermaterials. Die pharmazeutisch aktive Dosis liegt für Erwachsene in der Regel im Bereich von 150 mg bis 250 mg pro Kapsel und im Mittel bei etwa 200 mg, entsprechend der mittleren Dosis von etwa 3 mg/kg Körpergewicht.

Die Konsistenz der erfindungsgemässen Zubereitung ist nicht kritisch, da diese die Stabilität des Wirkstoffs praktisch nicht beeinflusst. Die Konsistenz der Zubereitung kann fest, pastös oder flüssig sein. Entscheidend für die Wahl des Gewichtsverhältnisses vom Wirkstoff zum Trägermaterial und für die Auswahl der verschiedenen Komponenten des Trägermaterials ist die industrielle Verarbeitbarkeit der Zubereitung im Kapsel-Abfüllverfahren. Auch darf kein Absetzen der Suspension bei der Abfüllung auftreten, das heisst, die Gehaltseinheitlichkeit der Zubereitung in der Kapsel während und nach der Abfüllung muss gewährleistet sein. Hierzu wird die Fliessfähigkeit der Zubereitung, welche in der Regel als Suspension vorliegt, so eingestellt, dass die Zubereitung während der Verarbeitung gut fliesst, jedoch nach der Verarbeitung im abgefüllten Zustand in der Kapsel als Paste vorliegt oder fest wird. Dies wird dadurch erreicht, dass im Abfüllverfahren die Zubereitung unter Druck und/oder leicht erhöhter Temperatur verarbeitet wird. Es ist auch möglich, der Zubereitung Zusatzstoffe beizufügen, wie beispielsweise feinverteiltes (disperses/hochdisperses) Aluminiumoxid oder disperses/hochdisperses Siliziumoxid), welche diese Verarbeitungsweise erleichtern. Die Herstellung einer solchen Konsistenz ist einfach und für den Fachmann problemlos.

Die Qualität und die Art der verwendeten Kapseln entsprechen den an sich bekannten im Handel erhältlichen Qualitäten. Die Herstellung von den erfindungsgemäss bevorzugten Weichgelatinekapseln ist z.B. in Pharmazeutische Technologie, H. Sucker, P. Fuchs, P. Speiser, Stuttgart 1991, Seiten 337-347 beschrieben.

Der Kapselüberzug dient dazu, die Gleitfähigkeit der rektalen Form zu verbessern und eine leichte Einführbarkeit in den Körper zu gewährleisten. Solche Gleitüberzüge sind an sich bekannt. An sich bekannten Überzugsmaterialien für Rektalkapseln sind beispielsweise Polyäthylenglykole mit durchschnittlichen Molekulargewichten von etwa 1'550 und 20'000, Glycerinmonooleat und Glycerindioleat, Polyvinylacetat und Talkum. So besteht eine an sich bekannte Zusammensetzung beispielsweise aus 40.5 Teilen Polyäthylenglykol mit einem durchschnittlichen Molekulargewicht von etwa 20'000, 17.4 Teilen Polyäthylenglykol mit einem durchschnittlichen Molekulargewicht von etwa 1'550, 26.0 Teilen Glycerinmonooleat und Glycerindioleat (Gemisch), 1.2 Teilen Polyvinylacetat und 14.9 Teilen Talkum. Kapselüberzüge sind beispielsweise in Pharmazeutische Technologie, H. Sucker, P. Fuchs, P. Speiser, Stuttgart 1991, Seite 343 beschrieben.

Für die Herstellung der erfindungsgemässen Zubereitung kann man beispielsweise so vorgehen, dass man die Trägermaterialien, beispielsweise das Hartfett, in einer beheizbaren und evakuierbaren Suspensionsprozessanlage bei Schmelztemperatur mischt. Der Schmelze werden anschliessend weitere Trägermaterialien, beispielsweise mittelkettige Triglyceride, sowie der Wirkstoff zugegeben werden, worauf die Schmelze zu einer homogenen Masse verarbeitet wird. Die erhaltene geschmolzene Suspension wird anschliessend durch ein Sieb filtriert. Hierauf wird das Filtrat wird durch Anlegen von Vakuum entgast und in Kapseln abgefüllt. Die derart erhaltenen Kapseln werden in an sich bekannter Weise mit einem Kapselüberzug versehen. Man kann auch ein anderes Vorgehen wählen, was für den Fachmann an sich problemlos ist.

Die weiteren Beispiele erläutern die Erfindung.

### Beispiel 1 (Herstellung einer erfindungsgemässen Zusammensetzung)

In einer beheizbaren und evakuierbaren Suspensionsprozessanlage aus Edelstahl mit eingebautem Rührer, Homogenisator und Umpumpsystem (Diessel-Werke) werden 30.6 kg Hartfett bei 45°C geschmolzen Anschliessend werden unter Rühren 44,4 kg mittelkettige Triglyceride und sodann 7.5 kg Artesunat eingearbeitet. Die Masse wird anschliessend unter Umpumpen während 10 Minuten homogenisiert. Sodann wird die Suspension unter mässigem Rühren bei 40°C +/- 2°C gehalten und durch ein Sieb mit 400µm Maschenweite gegeben. Die filtrierte Suspension wird durch Anlegen von Vakuum (Restdruck 0.5 bis 0.2 bar absolut) während 2 bis 3 Stunden entgast. Der erhaltene Ansatz wird gemäss Beispiel 10 in Weichgelatinekapseln abgefüllt. Die Charge genügt für 150'000 Kapseln à 50 mg.

### Beispiele 2 - 8

Die in Tabelle 1 aufgeführten Beispiele wurden analog zu Beispiel 1 hergestellt.

**Tabelle 1**

| Beispiel Nr. | Artesunat mg | Hartfett mg | mittelkettiges Triglycerid, mg | Additive |
|---|---|---|---|---|
| 2 | 200 | 40 | 350 | -.- |
| 3 | 200 | 20 | 570 | -.- |
| 4 | 50 | 32 | 359 | -.- |
| 5 | 50 | 4 | 387 | -.- |
| 6 | 400 | 15 | 175 | -.- |
| 7 | 200 | 21 | 369 | Oleum Arachidis, 35 mg |
| | | | | Stearinsäure, 10 mg |
| | | | | Aerosil, 3 mg |
| 8 | 200 | 21 | 369 | -.- |

### Beispiel 9 (Vergleichsbeispiel)

In einer beheizbaren und evakuierbaren Suspensionsprozessanlage aus Edelstahl mit eingebautem Rührer, Homogenisator und Umpumpsystem (Diessel-Werke) werden 25.2 kg Hartfett bei 45°C geschmolzen. Anschliessend werden 37.8 kg mittelkettige Triglyceride, 0.75 kg Sojalecithin und 12.75 kg Polyoxyäthylenglycol-tri-Ricinoleat zugegeben, homogen gemischt und mit 7.5 kg Artesunat versetzt. Die Masse wird anschliessend unter Umpumpen während 10 Minuten homogenisiert. Die Suspension wird unter mässigem Rühren bei 40°C +/- 2°C gehalten, und anschliessend durch ein Sieb (400µm Maschenweite) gegeben. Die filtrierte Suspension wird durch Anlegen von Vakuum (Restdruck 0.5 bis 0.2 bar absolut) während 2 bis 3 Stunden entgast. Der erhaltene Ansatz wird gemäss Beispiel 10 in Weichgelatinekapseln abgefüllt. Die Charge genügt für 150'000 Kapseln à 50 mg.

### Beispiel 10 (Herstellung von Weichgelatinekapseln)

(a) In einem separaten beheizbaren Edelstahlkessel mit Rührwerk (Diessel) werden 46 kg Gelatine, 19.8 kg Glycerin (85%) und 34.2 kg gereinigtes Wasser gegeben. Die Masse wird bei 70°C zu einer klaren Schmelze geschmolzen. Gasblasen werden durch langsames Rühren entfernt. Der Schmelze wird eine Suspension von 1.26 kg Titandioxid in Glycerin (85 %) (1 Teil Titandioxid auf 1 Teil Glycerin, 85%), und 0.14 kg Eisenoxid gelb in Glycerin 85% (1:2) in 0,7 kg gereinigtem Wasser zugegeben und bis zur homogenen Verteilung gerührt. Dieser Ansatz wird für mehrere Kapselchargen verwendet.
(b) Die Masse hergestellt gemäss Absatz (a) wird unter Kühlen in eine Kapsuliermaschine gebracht, und mit einer Zusammensetzung gemäss den Beispielen 1-8 nach dem Rotary-Die-Verfahren nach R.P. Scherer verkapselt. Die Kapseln werden in einem Rotationstrockner (System Scherer) vorgetrocknet, bis eine ausreichende Festigkeit vorhanden ist. Dann werden die Kapseln auf Horden weitergetrocknet. Die Zuluft beträgt hierbei 15 bis 26°C bei 10 - 40% relativer Luftfeuchte. Nach erfolgter Trocknung werden fehlerhafte Kapseln aussortiert.
(c) Für die Beschichtung der Kapseln mit dem Gleitüberzug wird ein Lochtrommelbeschichter (Glatt GC) verwendet. Die Lackformulierung wird unter Zwangs-Zu- und Abluft aufgesprüht, bis die erforderliche Menge pro Kapsel aufgebracht ist. Dazu werden pro Beschichtungsansatz 0.6675 kg Polyäthylenglykol mit einem durchschnittlichen Molekulargewicht 20'000, 0.2865 kg Polyäthylenglykol mit einem durchschnittlichen Molekulargewicht von 1550, 0.429 kg Gylcerinmonooleat und -dioleat (Gemisch), 0.0195 kg Polyvinylacetat und 0.2475 kg Talkum in einem Äthanol-Wasser Gemisch gelöst bzw. suspendiert. Die überzogenen Kapseln werden getrocknet und inspiziert. Fehlerhafte Kapseln werden aussortiert. Die Kapseln werden dann in PVDC-Aluminium-Durchdrückpackungen eingesiegelt.

### Stabilitätsprüfung der Zubereitungen

Die gemäss den obigen Beispielen 1-8 und Vergleichsbeispiel 9 hergestellten Zusammensetzungen wurden, abgefüllt in Rektalkapseln (hergestellt gemäss Beispiel 10), einer Stabilitätsprüfung unterworfen. Hierbei wurden folgenden Lagerbedingungen angewendet:
1. Bedingungen bei Raumtemperatur: 20-25°C, relative Feuchte 60%, Lagerdauer: 6 bis 24 Monate
2. im Trockenschrank:
   (i) 31°C, relative Feuchte: 75%, Lagerdauer: 6-24 Monate
   (ii) 40°C, relative Feuchte: 75%, Lagerdauer: 6-24 Monate
   (iii) 41°C, relative Feuchte: 75%, Lagerdauer: 6-24 Monate

Im Anschluss an die Lagerung wurden die folgenden Aspekte untersucht: das Aussehen der Zubereitung (Verfärbung etc.), die Durchschnittsmasse (Sollwert: 894 mg +/- 10%), die Füllmasse, die Zerfallszeit in Wasser bei 37°C, der Gehalt an Artesunat und als Abbauprodukte Dihydroartemisinin (Artesol) und Artemisinin. Es stellte sich heraus, dass bereits nach kurzer Zeit (d.i. nach 3 Monaten bei 40°C und 75% relativer Feuchte sowie nach 6 Monaten bei 31°C und 75% relativer Feuchte) eine hochgradige Instabilität der Vergleichszubereitung des Beispiels 9 auftrat, so dass die Untersuchungen nicht fortgesetzt wurden. Nach 3 Monaten bei 40°C und 75% relativer Feuchte sank der Gehalt an Artesunat auf 86.6%. Als Nebenprodukte wurden Artesol (6%) und Artemisinin (2.2%) gefunden. Analoge Analysenresultate zeigten sich nach 6 Monaten bei 31°C und 75% relativer Feuchte.

Demgegenüber wurde festgestellt, dass die Zubereitungen der Beispiel 1-8 eine ausgezeichnete Stabilität aufwiesen. Bei Raumtemperatur (20-25°C) und einer relativen Feuchte von 60% nach 13 Monaten war der Gehalt an Artesol und Artemisinin nicht messbar. Der Gehalt an Artesunat betrug unverändert 102.3%.

Im Trockenschrank bei 31°C, einer relativen Feuchte von 75% und einer Lagerung von 13 Monaten zeigte die Analyse die folgenden Werte: Artesol 0.5%, Artemisinin: nicht messbar, Artesunat: 100.3%.

Im Trockenschrank bei 40°C, einer relativen Feuchte von 75% und einer Lagerung von 13 Monaten zeigte die Analyse die folgenden Werte: Artesol 2.0%, Artemisinin: nicht messbar, Artesunat: 89.5%.

Gemäss diesen Resultaten kann die Stabilität der erfindungsgemässen Zusammensetzung auf 3 Jahre bei Raumtemperatur festgelegt werden.

## Patentansprüche

1. Zusammensetzung in Form von rektal anwendbaren Suppositorien, Rektalschäumen, Klistiere oder Rektalkapseln mit deutlich verbesserter Lagerstabilität, **dadurch gekennzeichnet, dass**:
(i) diese Zusammensetzung den Wirkstoff Artemisinin, Arteether, Artemether, Artemisin und/oder Artesunat, in einem gegenüber dem Wirkstoff inerten Trägermaterial enthält, wobei
(ii) dieses Trägermaterial eine Verbindung oder ein Gemisch von Verbindungen darstellt, ausgewählt aus der Gruppe umfassend: pflanzliche Wachse und Wachse tierischen Ursprungs mit Schmelzpunkten im Bereich von 47°C bis 88°C; Paraffinwachse mit Schmelzpunkten im Bereich von 47°C bis 65°C; mikrokristalline Wachse mit Schmelzpunkten im Bereich von 54°C und 105°C; Fette mit Schmelzpunkten im Bereich von 28°C bis 45°C und Öle mit Schmelzpunkten im Bereich von 0°C bis 20°C;
(iii) das Gewichtsverhältnis des Wirkstoffs zum Trägermaterial 5% bis 15%, bezogen auf das Gesamtgewicht des Trägermaterials, beträgt (ursprünglicher Anspruch 4), und
(iv) das Trägermaterial Verbindungen mit einem HLB-Wert von 7-9.9 in einer Konzentration von höchstens 2 Gew.-%, bezogen auf das Gewicht des Wirkstoffes, enthält.

2. Zusammensetzung nach Anspruch 1, **dadurch gekennzeichnet, dass** diese als Wirkstoff Artesunat, enthält.

3. Zusammensetzung nach Anspruch 1 oder 2, **dadurch gekennzeichnet, dass** das Gewichtsverhältnis des Wirkstoffs zum Trägermaterial 7% bis 12%, und vorzugsweise 8.5% bis 9.5%, bezogen auf das Gesamtgewicht des Trägermaterials, beträgt.

4. Zusammensetzung nach einem der Ansprüche 1-3, **dadurch gekennzeichnet, dass** keine Verbindung mit einem HLB-Wert von 7-9.9 in der Zusammensetzung anwesend ist.

5. Zusammensetzung nach einem der Ansprüche 1-4, **dadurch gekennzeichnet, dass** die Konzentration von Verbindungen, welche einen HLB-Wert von 4-6.9 und 10-13 aufweisen, höchstens 2 Gew.-%, bezogen auf das Gewicht des Wirkstoffes, beträgt.

6. Zusammensetzung nach Anspruch 5, **dadurch gekennzeichnet, dass** keine Verbindung mit einem HLB-Wert von 4-6.9 und 10-13 anwesend ist.

7. Zusammensetzung nach einem der Ansprüche 1-6, **dadurch gekennzeichnet, dass** die Konzentration von Verbindungen, welche einen HLB-Wert von 13.1-15 aufweisen, höchstens 2 Gew.-%, bezogen auf das Gewicht des Wirkstoffes, beträgt.

8. Zusammensetzung nach Anspruch 7, **dadurch gekennzeichnet, dass** keine Verbindung mit einem HLB-Wert von 13.1-15 anwesend ist.

9. Zusammensetzung nach einem der Ansprüche 1-.8 in der Form von Suppositorien oder Rektalkapseln.

10. Zusammensetzung nach Anspruch 9, **dadurch gekennzeichnet, dass** diese aus einer Weichgelatinekapsel besteht, welche den Wirkstoff gemäss Anspruch 1 enthält, wobei die Weichgelatinekapsel mit einem gleitfähigen Überzug versehen ist.

11. Verwendung der Zusammensetzung nach Anspruch 10 zur Herstellung eines Arzneimittels für die Bekämpfung von Malariaparasiten und multiresistenten Linien von plasmodium falciparum.

## Claims

1. Composition in the form of suppositories for rectal administration, rectal foams, enemas or rectal capsules of markedly improved storage stability, **characterized in that**:
(i) this composition contains the active ingredient artemisinin, arteether, artemether, artemisin and/or artesunate in an excipient that is inert towards the active ingredient,
(ii) said excipient being a compound or a mixture of compounds selected from the group comprising vegetable waxes and waxes of animal origin with melting points in the range 47°C to 88°C; paraffin waxes with melting points in the range 47°C to 65°C; microcrystalline waxes with melting points in the range 54°C to 105°C; fats with melting points in the range 28°C to 45°C; and oils with melting points in the range 0°C to 20°C;
(iii) the weight ratio of active ingredient to excipient is 5% to 15%, based on the total weight of the excipient (original Claim 4); and
(iv) the excipient contains compounds with an HLB of 7-9.9 in a concentration of at most 2% by weight, based on the weight of the active ingredient.

2. Composition according to Claim 1, **characterized in that** it contains artesunate as the active ingredient.

3. Composition according to Claim 1 or 2, **characterized in that** the weight ratio of active ingredient to excipient is 7% to 12%, preferably 8.5% to 9.5%, based on the total weight of the excipient.

4. Composition according to one of Claims 1 - 3, **characterized in that** no compound with an HLB of 7 - 9.9 is present in the composition.

5. Composition according to one of Claims 1- 4, **characterized in that** the concentration of compounds with an HLB of 4 - 6.9 and 10-13 is at most 2% by weight, based on the weight of the active ingredient.

6. Composition according to Claim 5, **characterized in that** no compound with an HLB of 4 - 6.9 and 10 -13 is present.

7. Composition according to one of Claims 1 - 6, **characterized in that** the concentration of compounds with an HLB of 13.1 -15 is at most 2% by weight, based on the weight of the active ingredient.

8. Composition according to Claim 7, **characterized in that** no compound with an HLB of 13.1 - 15 is present.

9. Composition according to one of Claims 1 - 8 in the form of suppositories or rectal capsules.

10. Composition according to Claim 9, **characterized in that** it consists of a soft gelatin capsule containing the active ingredient according to Claim 1, said soft gelatin capsule being provided with a lubricating coating.

11. Use of the composition according to Claim 10 for the preparation of a drug for combating malaria parasites and multiresistant lines of Plasmodium falciparum.

## Revendications

1. Composition en forme de suppositoires par voie rectale, de mousses rectales, de lavements ou capsules rectales avec une stabilité au stockage nettement améliorée, **caractérisée en ce que**
(i) cette composition comprend la substance active artémisinine, arteether, artemether, artémisine et/ ou artésunate, dans une matière porteuse qui est inerte par rapport à la substance active, où
(ii) cette matière porteuse est un composé ou un mélange de composés, choisi dans le groupe comprenant : des cires végétales et des cires d'origine animale avec des points de fusion compris entre 47°C et 88°C ; des cires paraffiniques avec des points de fusion compris entre 47°C et 65°C ; des cires microcristallines avec des points de fusion compris entre 54°C et 105°C ; des graisses avec des points de fusion compris entre 28°C et 45°C et des huiles avec des points de fusion compris entre 0°C et 20°C ;
(iii) la proportion de poids de la substance active par rapport à la matière porteuse est de 5% à 15%, par rapport au poids total de la matière porteuse (revendication 4 d'origine), et
(iv) la matière porteuse comprend des composés avec une valeur HLB de 7 - 9.9 dans une concentration d'au maximum 2 % en poids, par rapport au poids de la substance active.

2. Composition selon la revendication 1, **caractérisée en ce qu'**elle comprend comme substance active de l'artésunate.

3. Composition selon la revendication 1 ou 2, **caractérisée en ce que** la proportion en poids de la substance active par rapport à la matière porteuse est de 7% à 12%, et de préférence de 8.5% à 9.5%, par rapport au poids total de la matière porteuse.

4. Composition selon l'une quelconque des revendications 1 à 3, **caractérisée en ce qu'**aucun composé avec une valeur HLB entre 7 et 9.9 est présent dans la composition.

5. Composition selon l'une quelconque des revendications 1 à 4, **caractérisée en ce que** la concentration de composés ayant une valeur HLB de 4 - 6.9 à 10 - 13 s'élève au maximum à 2 % en poids, par rapport au poids de la substance active.

6. Composition selon la revendication 5, **caractérisée en ce qu'**aucun composé avec une valeur HLB de 4 - 6.9 à 10 -13 est présent.

7. Composition selon l'une quelconque des revendications 1 à 6, **caractérisée en ce que** la concentration de composés ayant une valeur HLB de 13.1 - 15 s'élève au maximum à 2 % en poids, par rapport au poids de la substance active.

8. Composition selon la revendication 7, **caractérisée en ce qu'**aucun composé avec une valeur HLB de 13.1 -15 est présent.

9. Composition selon l'une quelconque des revendications 1 à 8 en forme de suppositoires ou de capsules rectales.

10. Composition selon la revendication 9, **caractérisée en ce que** celle-ci est composée d'une capsule de gélatine souple qui contient la substance active selon la revendication 1, où la capsule de gélatine souple est munie d'un revêtement glissant.

11. Utilisation de la composition selon la revendication 10 pour la fabrication d'un médicament pour lutter contre des parasites de malaria et des lignes multirésistantes de plasmodium falciparum.
